(19) Europäisches Patentamt
European Patent Office
Office européen des brevets

(11) Veröffentlichungsnummer: **0 046 488**
**A2**

(12) # EUROPÄISCHE PATENTANMELDUNG

(21) Anmeldenummer: 81104999.8

(22) Anmeldetag: 27.06.81

(51) Int. Cl.[3]: C 07 D 493/08
C 07 D 317/20, C 07 D 317/24
C 07 D 319/06, A 23 K 1/16
A 23 L 1/30, A 23 J 3/00
C 12 N 1/00, A 61 K 31/335
A 61 K 31/365
//(C07D493/08, 319/00, 319/00),
(C07D493/08, 319/00, 317/00)

(30) Priorität: 28.07.80 DE 3028519
03.11.80 DE 3041295

(43) Veröffentlichungstag der Anmeldung:
03.03.82 Patentblatt 82/9

(84) Benannte Vertragsstaaten:
BE CH DE FR GB IT LI NL SE

(71) Anmelder: DYNAMIT NOBEL AKTIENGESELLSCHAFT
Patentabteilung Postfach 1209
D-5210 Troisdorf, Bez. Köln(DE)

(72) Erfinder: Schäfer, Gert, Dr.
Im Ardeytal 24
D-5810 Witten(DE)

(72) Erfinder: Hülsmann, Hans Leo, Dr.
Appendahl 42
D-5802 Wetter 4(DE)

(54) **Substituierte 2,5,7-Trioxa-bicyclo(2.2.2)octan-6-one, 3,6,8-Trioxa-bicyclo(3.2.1)octan-4-one sowie 1,3-Dioxan-2-carbonsäure- und 1,3-Dioxolan-2-carbonsäureverbindungen und Verfahren zu deren Herstellung.**

(57) Durch Reaktion von α-Ketocarbonsäuren der Formel R - CO - COOH oder deren Derivate, in denen R einen aliphatischen Rest mit 2 bis 18 Kohlenstoffatomen, einen araliphatischen Rest, besonders den Benzylrest oder einen aromatischen Rest bedeutet mit Glycerin oder Glyverinderivaten werden neue in 1-Stellung durch den Rest R substituierte 2,5,7-Trioxy-bicyclo [2.2.2] octan-6-one und in 5 Stellung durch den Rest R substituierte 3,6,8-Trioxa-bicyclo [3.2.1] octan-4-one hergestellt, die wieder in die Komponenten gespalten werden können.

EP 0 046 488 A2

Croydon Printing Company Ltd.

Troisdorf, den 18. Mai 1981
OZ: 80 042 / 077 Ausland

DYNAMIT NOBEL AKTIENGESELLSCHAFT
Troisdorf, Bez. Köln

Substituierte 2,5,7-Trioxan-bicyclo [2.2.2] octan-6-one, 3,6,8-Trioxa-bicyclo [3.2.1] octan-4-one sowie 1,3-Dioxan-2-carbonsäure- und 1,3-Dioxolan-2-Carbonsäureverbindungen und Verfahren zu deren Herstellung

Die Erfindung betrifft Produkte von neuer Struktur, die zwei bzw. drei biologisch aktive Komponenten im Molekül enthalten und als Zwischenprodukte zur Synthese pharmazeutisch wirksamer Substanzen verwendet werden können.

1-substituierte 2,5,7-Trioxa-bicyclo-[2.2.2] octan-6-one und 5-substituierte 3,6,8-Trioxa-bicyclo-[3.2.1] octan-4-one sind - mit Ausnahme der Methylverbindung (J. Gelas und A. Thiallier, Carbohydrate Res. 30 (1973) 21 )- bislang nicht bekannt.

Gegenstand der Erfindung sind 2,5,7-Trioxa-bicyclo-[2.2.2] octan-6-one der Formel

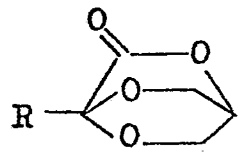

I

und 3,6,8-Trioxa-bicyclo [3.2.1] octan-4-one der Formel

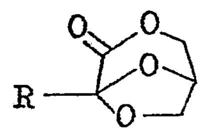

II

worin R einen verzweigten oder unverzweigten, gegebenenfalls mit weiteren Gruppen versehenen Alkylrest mit 2 bis 18 Kohlenstoffatomen, einen araliphatischen oder aromatischen Rest, der gegebenenfalls mit weiteren Gruppen substituiert ist, bedeutet sowie 1,3-Dioxan-2-carbonsäurederivate der Formel

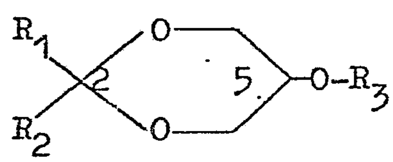

III

und 1,3-Dioxolan-2-carbonsäurederivate der Formel

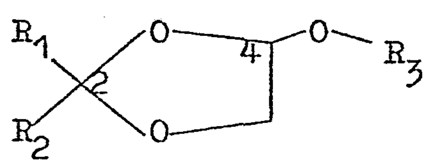

IV

worin $R_1$ einen verzweigten oder unverzweigten, gegebenenfalls mit weiteren Gruppen versehenen Alkylrest mit 1 bis 18, vorzugsweise mit 2 bis 8 Kohlenstoffatomen, einen araliphatischen oder aromatischen Rest, der gegebenenfalls mit weiteren Gruppen substituiert ist, $R_2$ eine Carboxylgruppe, deren Wasserstoffatom gegebenenfalls durch ein Metallatom ersetzt sein kann, oder eine Carbamoylgruppe und $R_3$ Wasserstoff oder den Carbonsäurerest einer physiologisch akzeptablen Carbonsäure oder den Carbonsäurerest eines pharmakologischen Wirkstoffs bedeuten.
Die neuen Stoffe sind im Gegensatz zu den α-Ketocarbonsäuren völlig stabil und unzersetzt lagerbar.

Als Alkylreste R und $R_1$ sind kurzkettiger Alkylrest mit 2 bis 8 Kohlenstoffatomen bevorzugt und sehr bevorzugt ein solcher Rest, der dem Rest R bzw. $R_1$ einer essentiellen Aminosäurevorstufe der Formel R-CO-COOH entspricht. Als araliphatischer Rest kommt vorzugsweise der Benzylrest und als aromatischer Rest kommt vorzugsweise der Phenylrest in Frage.
Weitere Gruppen in Alkylresten und aromatischen Resten können bei der Reaktion inerte Reste, besonders die in entsprechenden Aminosäuren vorkommenden Reste, beispielsweise die Methylmercaptogruppe des Methionins sein.

Als Rest $R_3$ kommt der Carbonsäurerest einer natürlichen Fettsäure mit 2 bis 18 Kohlenstoffatomen, wie beispielsweise Capryl-, Caprin-, Laurin-, Myristin-, Palmitin-, Stearin-, Öl , Linol-, Linolensäure, in Frage.
Als Rest $R_3$ kommt ferner der Carbonsäurerest eines pharmakologischen Wirkstoffs, besonders eines Pharmakons mit entzündungshemmender Wirkung, in Frage. Die am häufigsten beschriebenen Mittel mit entzündungshemmender Wirkung sind organische Säuren wie beispielsweise Acetylsalicylsäure, Indomethacin, 2-(3-Phenoxyphenyl)-propionsäure, und besitzen demnach eine Carboxylgruppe, die den Einbau des Wirkstoffmoleküls als Acylrest $R_3$ in die genannten 1,3-Dioxan- bzw. 1,3-Dioxolanverbindungen ermöglicht. Nach dem gleichen Prinzip sind auch Acylreste anderer Wirkstoffe, sofern diese eine veresterbare Carboxylgruppe aufweisen, als Rest $R_3$ möglich. Beispiele solcher pharmakologisch oder nutrativ wirksamer Carbonsäuren sind Aminosäuren, Ketosäuren, Carboxylgruppen enthaltende Bau-

BAD ORIGINAL

steine von Antibiotika, gegebenenfalls Di- und Polycarbonsäuren, Peptide etc.

Weiterer Gegenstand der Erfindung ist ein Verfahren zur Herstellung der Verbindungen der Formel I und II, dadurch, daß eine α-Ketocarbonsäure der Formel R-CO-COOH, in der R die angegebene Bedeutung besitzt, oder deren Ester, Ketale, Ketalester, Halogenide oder vergleichbare Derivate mit Glycerin oder einem den Glycerinrest liefernden Derivat bei einer eine ausreichende Reaktionsgeschwindigkeit auslösenden Temperatur unterhalb 160°C und in Anwesenheit von sauren Katalysatoren umgesetzt wird.

Die Herstellung der Substanzen erfolgt im allgemeinen durch Umsetzung einer α-Ketocarbonsäure der Formel R-CO-COOH mit Glycerin unter Bedingungen, welche die Wasserabspaltung aus den funktionellen Gruppen der beiden Reaktionspartner fördern, wobei die Ketogruppe der α-Ketocarbonsäure mittels zweier Hydroxylgruppen des Glycerins in das entsprechende Ketal und die Carboxylgruppe der Säure mittels der dritten Hydroxylgruppe des Glycerins in den entsprechenden Ester überführt wird. Zur Beschleunigung der Reaktionsgeschwindigkeit kann die Umsetzung bei erhöhter Temperatur vorgenommen werden. Eine obere Grenze ist jedoch durch die Labilität der α-Ketocarbonsäure gegeben. So sind Reaktionstemperaturen oberhalb von ca. 160°C zu vermeiden, da sonst zunehmend Zersetzungs- und Polymerprodukte entstehen. Ausreichende Reaktionsgeschwindigkeiten erhält man im allgemeinen im Temperaturbereich von 20 bis 120°C.
Der Reaktionsdruck liegt bevorzugt bei Normaldruck, jedoch ist auch verminderter Druck oder Überdruck von 1 mbar bis 10 bar möglich.

Das entstehende Reaktionswasser wird zweckmäßig, sofern die Flüchtigkeit der Reaktionspartner dies erlaubt, durch Abdestillieren bei Normaldruck oder unter vermindertem Druck entfernt.

Die Molverhältnisse der Reaktionspartner liegen allgemein im Verhältnis 1 : 1, jedoch ist der Überschuß eines Partners bis 200 % möglich, im allgemeinen aber nicht zweckmäßig.

Anstelle der Ketocarbonsäuren können deren Derivate welche die Gruppierung 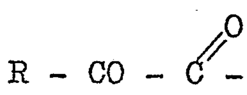 oder 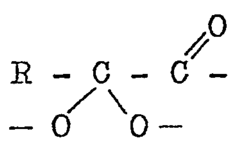 bei der Reaktion besteuern, beispielsweise die Ketale d.h. Acetale der Ketocarbonsäuren, besonders solche mit Acetalgruppen von 1 bis 3 Kohlenstoffatomen, die entsprechenden Ketalester mit Estergruppe von bevorzugt 1 bis 8, sehr bevorzugt 1 bis 3 Kohlenstoffatomen oder Halogenide, besonders Chloride der α-Ketocarbonsäuren, sowie weitere die genannte Gruppierung liefernde Derivate Verwendung finden.

Auch anstelle des Glycerins können Glycerinderivate, die den Glycerinrest in verkappter Form enthalten, die trifunktionelle Gruppe $-H_2C-\underset{|}{CH}-CH_2-$ bzw. diese Gruppe zusammen mit ein bis drei daran gebundenen Sauerstoffbrücken beisteuern, beispielsweise Glycerinäther bzw. Glycerinester wie Isopropylidenglycerin, Epoxide wie Glycid oder Epichlorhydrin oder weitere die genannte trifunktionelle Gruppe beisteuernde Glycerinderivate Verwendung finden.

Dabei braucht die jeweilige Sauerstoffbrücke - O - jeweils nur von einem der Reaktionspartner geliefert zu werden, so daß der andere Reaktionspartner z.B. eine abspaltbare Gruppe wie Halogene aufweisen kann.

BAD ORIGINAL

Die Durchführung der Cyclisierungsreaktion wird ferner durch den Einsatz von Katalysatoren erleichtert. Geeignet sind saure Katalysatoren, welche sowohl die Ketalisierungswie auch die Veresterungsreaktion günstig beeinflussen. Bewährt haben sich beispielsweise Schwefelsäure, Alkyl- oder Arylschwefelsäuren wie p-Toluolsulfonsäure, Phosphorsäure. Die Konzentration der Katalysatoren kann 0,01 bis 3 Gew.-%, vorzugsweise 0,05 bis 1 Gew.-%, bezogen auf die Menge der beiden Reaktionspartner, betragen.

Die Reaktion kann in einem inerten Lösungsmittel, wobei es nicht erforderlich ist, daß die Reaktionspartner vollkommen gelöst sind, oder aber auch ohne jede Verwendung eines Lösungsmittels durchgeführt werden. Im Falle der Verwendung eines Lösungsmittels kann es zweckmäßig sein, dieses als Schleppmittel zum Ausdestillieren des Reaktionswassers zu benutzen, um die Bildung des cyclischen Ketalesters zu beschleunigen.
Geeignete inerte Lösungsmittel sind beispielsweise aromatische Kohlenwasserstoffe wie Benzol oder Toluol, chlorierte aliphatische Kohlenwasserstoffe wie Tetrachlorkohlenstoff, Chloroform, Methylenchlorid, Trichloräthan sowie beispielsweise Dimethylformamid.
Soweit Derivate der Ketocarbonsäuren bzw. des Glycerins z.B. wegen leichterer Zugänglichkeit Verwendung finden, werden beispielsweise durch Abspaltung entstehende Alkohole oder Halogenwasserstoffsäuren während oder nach der Reaktion zweckmäßig entfernt, beispielsweise durch Destillation.

In einer weiteren, besonders schonenden Variante des Verfahrens kann auf das Entfernen des Reaktionswassers ganz verzichtet werden. In diesem Fall ist jedoch der Einsatz eines wasserbindenden Mittels, beispielsweise von Thionylchlorid, zweckmäßig. Unter diesen Bedingungen verläuft die Umsetzung auch schon bei Raumtemperatur mit genügender Geschwindigkeit.

Bei der Herstellung der erfindungsgemäßen Produkte nach einer der beschriebenen Verfahrensvarianten wird zumeist ein Gemisch aus dem Isomerenpaar 2,5,7-Trioxa [2.2.2] octan-6-on und 3,6,8-Trioxa-bicyclo [3.2.1] octan-4-on erhalten, das auch gemeinsam verwendet werden kann. Das Gewichtsverhältnis der beiden Isomeren wird zwar durch die Struktur der eingesetzten α-Ketocarbonsäure beeinflußt, überwiegend entsteht jedoch das symmetrische 2,5,7-Trioxa [2.2.2] octan-6-on. Durch Auftrennung des Gemischs können gegebenenfalls auch die reinen Komponenten isoliert werden. Als Trennverfahren kommen zu diesem Zweck vorzugsweise fraktionierte Kristallisation oder Destillation sowie chromatographische Trennmethoden, die den jeweiligen Gegebenheiten entsprechend anzupassen sind, in Betracht. Durch fraktionierte Kristallisation aus geeigneten Lösungsmitteln läßt sich im allgemeinen schon nach einmaliger Kristallisation das symmetrisch gebaute, gut kristallierende Isomere vom Typ des 2,5,7-Trioxa-bicyclo [2.2.2] octan-6-on in genügender Reinheit erhalten. Das weniger gut kristallisierende, isomere 3,6,8-Trioxa-bicyclo [3.2.1] octan-4-on kann anschließend aus den eingedampften Mutterlaugen, gegebenenfalls auch nach Abtrennung schwerer flüchtiger Begleitstoffe nach Überdestillieren des Rohprodukts, kristallin erhalten und schließlich durch Umkristallisieren gereinigt werden. Trennungen der beiden Isomeren sind beispielsweise auch an mit Kieselgel beschichteten Platten oder durch Säulenchromatographie mit Kieselgel als stationärer Phase möglich.

Weiterer Gegenstand der Erfindung ist ein Verfahren zur Herstellung der Verbindungen III und IV gemäß einem oder mehreren der Ansprüche 1 bis 6, welches dadurch gekennzeichnet ist, daß

2,5,7-Trioxa-bicyclo[2.2.2] octan-6-one der Formel

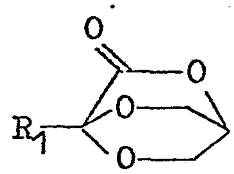

I oder/und

3,6,8-Trioxabicyclo [3.2.1] octan-4-one der Formel

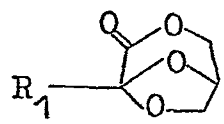

II

in 1,3-Dioxan-2-carbonsäure-Derivate der Formel

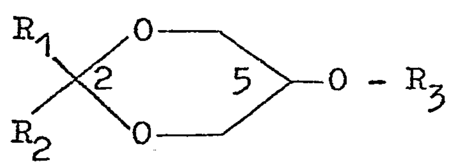

III

bzw. 1,3-Dioxolan-2-carbonsäure-Derivate der Formel

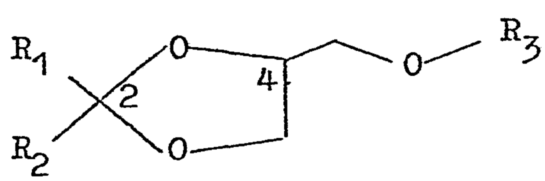

IV

umgewandelt werden, in denen $R_1$, $R_2$ und $R_3$ die oben angegebene Bedeutung haben,

a) durch Behandlung mit basisch reagierenden Verbindungen, vorzugsweise mit Alkalihydroxiden, oder

b) durch Behandlung mit basisch reagierenden Verbindungen, vorzugsweise mit Alkalihydroxiden, und nachfolgender Freisetzung der Oxycarbonsäure mittels Mineralsäure oder

BAD ORIGINAL

c) durch Behandlung mit Ammoniak oder

d) durch Umsetzung eines nach a), b) oder c) erhaltenen Produktes mit einer physiologisch akzeptablen Carbonsäure bzw. einem geeigneten Carbonsäurederivat oder mit einem eine Carboxylgruppe aufweisenden pharmakologischen Wirkstoff bzw. einem entsprechenden Derivat des Wirkstoffs.

Die Herstellung der erfindungsgemäßen Substanzen erfolgt in zwei Stufen, soweit $R_3$ ein Carbonsäurerest ist.

In der ersten Verfahrensstufe wird ein in 1-Stellung durch den Rest $R_1$ substituiertes 2,5,7-Trioxa-bicyclo [2.2.2] octan-6-on oder ein in 5-Stellung substituiertes 3,6,8-Trioxa-bicyclo[3.2.1]octan-4-on, in denen $R_1$ die oben angegebene Bedeutung besitzt und deren Herstellung in der deutschen Patentanmeldung P 30 28 519.9 beschrieben ist, mit basisch reagierenden Verbindungen, vorzugsweise mit Alkalihydroxiden oder Ammoniak, umgesetzt. Damit läßt sich überraschend eine selektive Spaltung des Lactonrings unter Erhalt der Ketalgruppierung erreichen, wobei aus den Bicyclo-[2.2.2]octan-6-onen die entsprechenden 1,3-Dioxan-2-carbonsäurederivate mit einer freien Hydroxylgruppe in 5-Stellung und aus den Bicyclo [3.2.1]octan-4-onen die entsprechenden 1,3-Dioxolan-2-carbonsäurederivate mit einer Hydroxymethylgruppe in 4-Stellung entstehen. Die Lactonspaltung läßt sich im allgemeinen bereits bei Raumtemperatur durchführen. Die Anwendung höherer Temperaturen bis etwa 60°C zur Beschleunigung der Umsetzung ist ebenfalls möglich.
Die basischen Verbindungen sind in äquivalenter Menge oder im Überschuß bis etwa 100 Mol % zu verwenden.
Im Falle der Verwendung von Alkalihydroxiden entstehen 1,3-Dioxan-2-carbonsäurederivate bzw. 1,3-Dioxolan-2-car-

BAD ORIGINAL

bonsäurederivate der oben angegebenen Formel, in der $R_2$ eine Carboxylgruppe, deren Wasserstoffatom durch ein Alkalimetallatom ersetzt ist, und $R_3$ ein Wasserstoffatom bedeutet. Die entsprechenden freien Säuren lassen sich aus diesen Salzen in bekannter Weise durch Ansäuern mittels mindestens äquivalenten Mengen von Mineralsäuren in Freiheit setzen und isolieren. Im Falle der Verwendung von Ammoniak, welches durch Einleiten von gasförmigem Ammoniak in die wasserfreie Lösung der Bicyclo[2.2.2]octan-6-one bzw. Bicyclo [3.2.1]octan-4-one oder aber auch durch Behandlung der Ausgangsverbindungen mit wäßriger Ammoniaklösung erfolgen kann, werden direkt die entsprechenden 1,3-Dioxan-2-carbonsäureamide bzw. 1,3-Dioxolan-2-carbonsäureamide erhalten, wobei in diesem Fall in den oben angegebenen Formeln $R_2$ eine Carbamoylgruppe und $R_3$ ein Wasserstoffatom bedeutet.

Als weitere basische Verbindungen kommen Alkalialkoholate, gegebenenfalls auch Erdalkalialkoholate oder Erdalkalihydroxide ,in Frage.

In der zweiten Verfahrensstufe wird ein nach der oben angegebenen Methode erhaltenes 1,3-Dioxan-2-carbonsäurederivat bzw. ein 1,3-Dioxolan-2-carbonsäurederivat, vorzugsweise ein 1,3-Dioxan-2-carbonsäureamid bzw. ein 1,3-Dioxolan-2-carbonsäureamid, mit einer Fettsäure oder einem Pharmakon, das eine Carboxylgruppe im Molekül aufweist, vorzugsweise in Form der entsprechenden Säurechloride, umgesetzt.

Die Reaktion erfolgt bei 0 bis etwa 60°C bei Normaldruck oder gegebenenfalls Überdruck bis etwa 2 bar.

Gegebenenfalls können Mischungen von Dioxan- und Dioxolan-Derivaten mit gleichen Substituenten $R_1$, $R_2$ und $R_3$ hergestellt werden, sofern die Ausgangsstoffe nicht in die Isomeren getrennt werden.

BAD ORIGINAL

Nach einer bevorzugten Ausführungsform des Verfahrens wird ein 1,3-Dioxan-2-carbonsäureamid bzw. ein 1,3-Dioxolan-2-carbonsäureamid in einem inerten Lösungsmittel, beispielsweise einem chlorierten aliphatischen Kohlenwasserstoff wie Tetrachlormethan, Trichloräthan oder Chloroform, das einen kleineren Anteil Pyridin als Katalysator enthält, oder auch in wasserfreiem Pyridin ohne weitere Zusätze gelöst oder suspendiert. Der Lösung bzw. Suspension wird unter Kühlung die äquivalente Menge des betreffenden Säurechlorids über einen Zeitraum hin tropfenweise zugegeben. Nachdem einige Stunden bei 0°C gerührt worden ist, wird die Mischung noch einige Zeit auf Raumtemperatur bzw. eine höhere Temperatur, vorzugsweise bis höchstens 60°C gebracht und dann in bekannter Weise aufgearbeitet.
Bei Verwendung eines Säureanhydrids wie Acetanhydrid erfolgte die Zugabe bei Zimmertemperatur und die Reaktion bei bis zu 60 °C.
Nach Isolierung und Umkristallisieren aus geeigneten Lösungsmitteln wird das gebildete Produkt der oben dargestellten Formel, in der $R_1$, $R_2$ und $R_3$ die angegebene Bedeutung besitzen, in ausgezeichneter Reinheit erhalten.

Es ist ferner möglich, anstelle der Säurechloride andere reaktionsfähige Carbonsäurederivate, beispielsweise die entsprechenden Carbonsäureanhydride mit den 1,3-Dioxan-2-carbonsäureamiden bzw. mit den 1,3-Dioxolan-2-carbonsäureamiden umzusetzen, welche einen Überschuß, im allgemeinen die 2- bis 5-fache Molmenge erfordern.

Weiterhin ist es möglich, die 1,3-Dioxan-2-carbonsäureamide bzw. 1,3-Dioxolan-2-carbonsäureamide auch mit freien Carbonsäuren zu verestern. In diesem Fall ist es je-

BAD ORIGINAL

doch erforderlich, unter Beibehaltung der oben beschriebenen Temperaturgrenze ein wasserabspaltendes Reagenz wie beispielsweise Dicyclohexylcarbodiimid einzusetzen.

Dagegen ist es nicht möglich, die freien Hydroxylgruppen der Ausgangsstoffe durch direkte Veresterung mit Carbonsäuren nach den üblichen Methoden bei erhöhter Temperatur unter Wasserabspaltung in Anwesenheit von sauren oder basischen Katalysatoren in die entsprechenden Estergruppierungen zu überführen, da unter diesen Bedingungen eine Umkehrung der Lactonspaltungsreaktion unter Bildung der Bicyclo [2.2.2] octan-6-one bzw. Bicyclo [3.2.1] octan-4-one erfolgt.

Mit dem erfindungsgemäßen Verfahren wird ein Verfahren bereitgestellt, das es erlaubt, die als instabil und oxidationsempfindlich bekannten α-Ketocarbonsäuren der Formel $R_1$-CO-COOH, vorzugsweise solche, die als Vorstufen der natürlichen Aminosäuren im Stoffwechsel bedeutsam sind, in eine haltbare und oxidationsstabile Form zu überführen. Die erfindungsgemäßen Produkte eröffnen die Möglichkeit, die Wirkungsprinzipien von mindestens zwei, bevorzugt von drei verschiedenen biologisch aktiven Substanztypen in ein und demselben Molekül zu verknüpfen, nämlich den α-Ketocarbonsäurerest, der im Stoffwechsel in die entsprechende Aminosäure umgewandelt werden kann, den Rest des Glycerins, das in enger Beziehung zum Kohlenhydratstoffwechsel steht und den Rest einer natürlichen Fettsäure bzw. den Rest eines Pharmakons mit vorzugsweise entzündungshemmender Wirkung.

Demgemäß ist schließlich ein weiterer Gegenstand der Erfindung die Verwendung der erfindungsgemäßen Stoffe als Arzneimittel der Human- und Tiermedizin im Rahmen des Aminosäurestoffwechsels, als Transportform von carboxyl-

1 gruppenhaltigen Pharmaka sowie als Ernähungsbestandteil für Mensch, Tier und Mikroorganismen.
Es ist von besonderem Vorteil, daß Ketocarbonsäuren im Organismus vollständig in die entsprechende L-Aminosäure umgesetzt werden, womit die bei der direkten Gabe von Aminosäuren verbundenen Stoffverluste durch Racemattrennung vermieden sind.
Die erfindungsgemäßen Produkte sind wertvolle Zwischenprodukte für die Synthese pharmazeutisch wirksamer Substanzen.

Dr.La/Ra

1 <u>Beispiel 1</u>

1-Benzyl-2,5,7-trioxa-bicyclo [2.2.2] octan-6-on

Eine Mischung aus 41 g (0,25 Mol) Phenylbrenztraubensäure, 27,5 g (0,3 Mol) Glycerin, 0,5 g p-Toluolsulfonsäure und 50 ml Benzol wurde unter Stickstoff allmählich auf 90 bis 100°C aufgeheizt. Nach Abdestillieren des Lösungsmittels wurde der Druck im Reaktionsgefäß auf ca. 20 mbar erniedrigt. Die Temperatur der Reaktionsmischung wurde während des Überdestillierens des Reaktionswassers unter intensivem Rühren für 2 Std. auf 120°C gehalten. Dabei wurde die anfangs inhomogene Mischung gegen Ende der Reaktion transparent. Nach Beendigung der Umsetzung wurde der Kolbeninhalt auf ca. 60°C gekühlt und mit 100 ml Ethanol versetzt. Nach weiterem Abkühlen und Zugabe von 30 ml Diethylether wurde bei ca. 0°C durchkristallisiert.
Es wurden 29 g 1-Benzyl-2,5,7-trioxa-bicyclo [2.2.2] octan-6-on, entsprechend einer Ausbeute von 53 %, in Form farbloser Kristalle vom Schmelzpunkt 166°C ( aus Ethanol ) erhalten.

| Kennzahlen: | SZ | VZ | OHZ |
|---|---|---|---|
| berechnet | 0 | 254 | 0 |
| gefunden | 0,2 | 252 | 0 |

IR (KBr): 3530, 1775 (Lacton gespannt), 1225/1185, 1095/1080 $cm^{-1}$

$^1$H-NMR (DMSO-$d_6$): $\delta$ = 3,09 (s, 2 H ); 4,05/5,06 ($A_2B_2X$-System, 4 H/ 1 H ); 7,25 ( m, 5 H )

Massenspektrum: m/e 220 ($M^+$)

Elementranalyse:

| | | C | H |
|---|---|---|---|
| berechnet | ($C_{12}H_{12}O_4$) | 65,45 | 5,49 |
| gefunden | | 65,60 | 5,62 |

Beispiel 2

Die Cyclisierungsreaktion nach Beispiel 1 wurde wiederholt, wobei anstelle der Phenylbrenztraubensäure die äquivalente Menge Phenylbrenztraubensäure-ethylester eingesetzt wurde. Als Reaktionsprodukt wurde 1-Benzyl-2,5,7-trioxa-bicyclo [2.2.2] octan-6-on in vergleichbarer Ausbeute und Reinheit erhalten.

Beispiel 3

Die Cyclisierungsreaktion nach Beispiel 1 wurde mit der äquivalenten Menge Isopropylidenglycerin anstelle von Glycerin durchgeführt. Als Reaktionsprodukt wurde 1-Benzyl-2,5,7-trioxa-bicyclo [2.2.2] octan-6-on in vergleichbarer Ausbeute und Reinheit erhalten.

Beispiel 4

5-Benzyl-3,6,8-trioxa-bicyclo [3.2.1] octan-4-on

Die bei der Herstellung von 1-Benzyl-2,5,7-trioxa-bicyclo [2.2.2] octan-6-on nach Beispiel 1 angefallenen Mutterlaugen wurden eingedampft. Der Rückstand wurde in Chloroform aufgenommen und nach Abtrennung des ausgeschiedenen Glycerins und Neutralisation mit festem Natriumhydrogencarbonat einer Kurzwegdestillation bei 0,3 mbar unterworden. Der Rückstand wurde soweit wie möglich - die Kopftemperatur stieg gegen Ende der Destillation bis auf 200°C - ausdestilliert. Es wurde ein intensiv gelb gefärbtes Destillat erhalten, das bereits an den Kühlerflächen teilweise erstarrte. Es wurden 14 g 5-Benzyl,3,6,8-trioxa-bicyclo [3.2.1] octan-4-on (Racemat), entsprechend einer Ausbeute von 26 % erhalten, das nach Reinigung in Form farbloser Kristalle vom Schmelzpunkt 142-143°C (aus Ethanol)anfiel.

Das gleiche Produkt wurde in geringer Ausbeute auch bei der Umsetzung von Phenylbrenztraubensäure mit Glycerin nach Beispiel 1, jedoch ohne die Verwendung von p-Toluol-

BAD ORIGINAL

sulfonsäure als Katalysator, anstelle des isomeren 1-Benzyl-2,5,7-trioxa-bicyclo [2.2.2] octan-6-on erhalten.

| Kennzahlen: | SZ | VZ | OHZ |
|---|---|---|---|
| berechnet | 0 | 254 | 0 |
| gefunden | 1,8 | 250 | 0 |

IR(KBr): 1745, 1235, 1200/1180, 1100/1070 $cm^{-1}$

$^1$H-NMR (DMSO-$d_6$): $\delta$ = 3,27 (AB-System, 2 H); 3,95/4,92/4,47 (ABX/A'B'X-System, 2H/1H/2H); 7,29 (m, 5H)

Massenspektrum: m/e 220 ($M^+$)

Elementaranalyse:

| | | C | H |
|---|---|---|---|
| berechnet | ($C_{12}H_{12}O_4$) | 65,45 | 5,49 |
| gefunden | | 65,98 | 5,66 |

<u>Beispiel 5</u>

1-Isopropyl-2,5,7-trioxa-bicyclo [2.2.2] octan-6-on

Eine Mischung aus 116 g (1 Mol Dimethylbrenztraubensäure) und 101 g (1,1 Mol) Glycerin in 275 ml abs. Ether wurde unter Rühren bei 0°C tropfenweise (Dauer ca. 1 Std.) mit 170 g (1,4 Mol) Thionylchlorid versetzt. Nach 5 Std. Rühren bei Raumtemperatur wurde das Lösungsmittel und der Überschuß an Thionylchlorid abdestilliert. Der Rückstand wurde in 200 ml Methanol aufgenommen und mit festem Natriumhydrogencarbonat neutral gestellt. Nach Abdestillieren des Lösungsmittels wurde im Vakuum fraktioniert. Neben einer geringen Menge an unveränderter Dimethylbrenztraubensäure fielen in der Hauptfraktion 94 g 1-Isopropyl-2,5,7-trioxa-bicyclo [2.2.2] octan-6-on, entsprechend einer Ausbeute von 55 %, im Siedebereich 82-89°C/,02-0,25 mbar an. Das flüssige Produkt wurde in wenig Diethylether/Methanol (6:1) aufgenommen und mit

BAD ORIGINAL

Petrolether versetzt. Nach längerem Aufbewahren bei -10°C wurde reines 1-Isopropyl-2,5,7-trioxa-bicyclo [2.2.2] octan-6-on in Form farbloser Kristalle vom Schmelzpunkt 40-41°C erhalten.

$^1$H-NMR ($CCl_4$): $\delta$ = 0,88 (d, 6H); 1,99 (m, 1H); 3,93/4,61 (AA'BB'X-System, 4H/1H)

Massenspektrum: m/e 144 ($M^+$-28)

Elementaranalyse:

| | | C | H |
|---|---|---|---|
| berechnet | ($C_8H_{12}O_4$) | 55,81 | 7,03 |
| gefunden | | 55,72 | 6,82 |

Beispiel 6

1-sec-Butyl-2,5,7-trioxa-bicyclo [2.2.2] octan-6-on

Methylethylbrenztraubensäure wurde analog Beispiel 5 mit Glycerin umgesetzt. Beim Aufarbeiten des Reaktionsprodukts wurde 1-sec-Butyl-2,5,7-trioxa-bicyclo [2.2.2] octan-6-on als wasserklares Destillat (Siedebereich 88-99°C/0,5 mbar) in vergleichbarer Ausbeute erhalten. Das flüssige Produkt wurde in wenig Diethylether/Methanol (50:1) aufgenommen und mit Petrolether versetzt. Nach längerem Aufbewahren bei -10° wurde reines 1-sec-Butyl-2,5,7-trioxa-bicyclo [2.2.2] octan-6-on in Form farbloser Kristalle vom Schmelzpunkt 60-61°C erhalten.

$^1$H-NMR ($CDCl_3$): $\delta$ = 0,84-2,06 (m, 9H); 4,08-4,72 (AA'BB'X-System, 5H)

Elementaranalyse:

| | | C | H |
|---|---|---|---|
| berechnet | ($C_9H_{14}O_4$) | 58,05 | 7,58 |
| gefunden | | 58,12 | 7,44 |

BAD ORIGINAL

Beispiel 7

1-Isobutyl-2,5,7-trioxa-bicyclo [2.2.2] octan-6-on

Isopropylbrenztraubensäure wurde analog Beispiel 5 mit Glycerin umgesetzt. Nach entsprechender Aufarbeitung wurde reines 1-Isobutyl-2,5,7-trioxa-bicyclo [2.2.2] octan-6-on in Form farbloser Kristalle vom Schmelzpunkt 54-55°C erhalten.

Massenspektrum: m/e 142 ($M^+$-44 )

Elementaranalyse:

| | | C | H |
|---|---|---|---|
| berechnet | ($C_9H_{14}O_4$) | 58,05 | 7,58 |
| gefunden | | 57,95 | 7,74 |

Beispiel 8

5c-Hydroxy-2-benzyl-[1,3]dioxan-2r-carbonsäureamid

Eine Mischung aus 11 g (0,05 Mol) 1-Benzyl-2,5,7-trioxa-bicyclo [2.2.2] octan-6-on, 130 ml Ethanol und 55 ml einer 25 Gew.-%igen Ammoniaklösung wurde 15 Min. auf 60°C erwärmt. Die Lösung wurde 24 Std. bei Raumtemperatur aufbewahrt und danach am Rotationsverdampfer (40 °C; 50 mbar) auf ca. ein Drittel des Volumens eingedampft. Nach dem Abkühlen wurden 10 g 5c-Hydroxy-2-benzyl-[1,3]dioxan-2r-carbonsäureamid, entsprechend einer Ausbeute von 92 %, in Form farbloser Kristalle vom Schmelzpunkt 183 - 184°C ( aus Ethanol ) erhalten.

$^1$H-NMR (DMSO-$d_6$): $\delta$ = 2,94 (s, 2H); 3,14-3,91 (m, 5H); 5,03 (d, 1H); 7,22 (m, 5H); 7,37 (breites s, 2H)

Massenspektrum: m/e 193 ($M^+$-44)

Elementaranalyse:

| | C | H | N |
|---|---|---|---|
| berechnet ($C_{12}H_{15}NO_4$) | 60,75 | 6,37 | 5,90 |
| gefunden | 60,59 | 6,48 | 5,69 |

BAD ORIGINAL

Beispiel 9

In Abwandlung von Beispiel 8 wurde in eine Lösung von 11g 1-Benzyl-2,5,7-trioxa-bicyclo [2.2.2] octan-6-on in 130 ml wasserfreiem Ethanol 5 g Ammoniakgas eingeleitet. Die gesättigte Lösung wurde 24 Std. bei Raumtemperatur aufbewahrt und dann gemäß Beispiel 8 aufgearbeitet. Als Reaktionsprodukt wurde 5c-Hydroxy-2-benzyl- [1,3] dioxan-2r-carbonsäureamid in vergleichbarer Ausbeute und Reinheit erhalten.

Beispiel 10

5c-Hydroxy-2-benzyl- [1,3] dioxan-2r-carbonsäure

Eine Lösung von 2,2 g (0,01 Mol) 1-Benzyl-2,5,7-trioxa-bicyclo [2.2.2] octan-6-on in 100 ml 1n ethanolischer Kalilauge wurde 24 Std. bei Raumtemperatur aufbewahrt. Nach Abdampfen von ca. 90 ml des Lösungsmittels wurde das entstandene Kaliumsalz mit 40 ml 10 Gew.-%iger Salzsäure umgesetzt. Es wurden 1,7 g 5c-Hydroxy-2-benzyl- [1,3] dioxan-2r-carbonsäure, entsprechend einer Ausbeute von 71 %, in Form farbloser plattenförmiger Kristalle vom Schmelzpunkt 142°C (aus Wasser) erhalten.

Kennzahlen: SZ berechnet 236; gefunden 238
IR (KBr) : 3300, 2995, 1710, 1200/1185, 1070/1060 $cm^{-1}$

Beispiel 10 a

Das nach Beispiel 10 entstandene Kaliumsalz entsprach bei der Elementaranalyse und massenspektrographisch dem K-Salz der Carbonsäure nach Beispiel 10.

BAD ORIGINAL

Beispiel 11

4c-Hydroxymethyl-2-benzyl- [1,3]dioxolan-2r-carbonsäureamid

Eine Mischung aus 2,2 g (0,01 Mol) 5-Benzyl-3,6,8-trioxa-bicyclo [3.2.1]-octan-4-on, 25 ml Ethanol und 10 ml 25 Gew.-%iger Ammoniaklösung wurde auf 60°C erwärmt bis eine klare Lösung entstanden war. Nach 24 Std. Aufbewahren bei Raumtemperatur wurde am Rotationsverdampfer (40 °C 50 mbar)auf die Hälfte eingeengt.
Der Rückstand wurde mit 100 ml Diethylether versetzt und bei 0°C zum Kristallisieren gebracht. Es wurden 1,5 g 4c-Hydroxymethyl-2-benzyl-[1,3]-dioxolan-2r-carbonsäureamid, entsprechend einer Ausbeute von 63 %, in Form farbloser Kristalle vom Schmelzpunkt 130-131°C erhalten.

Elementaranalyse:

| | C | H | N |
|---|---|---|---|
| berechnet ($C_{12}H_{15}NO_4$) | 60,75 | 6,37 | 5,90 |
| gefunden | 60,46 | 6,26 | 5,68 |

Beispiel 12

4c-Hydroxymethyl-2-benzyl- [1,3]dioxolan-2r-carbonsäure

2,2 g (0,01 Mol) 5-Benzyl-3,6,8-trioxa-bicyclo [3.2.1]-octan-4-on wurden in 50 ml 1n ethanolischer Kalilauge 10 Min. zum Sieden erhitzt. Die klare Lösung wurde bei Normaldruck auf 5 ml eingedampft.
Der Rückstand wurde in 50 ml 2n Salzsäure aufgenommen und durch Kühlen auf 0°C zum Kristallisieren gebracht. Es wurden 1,9 g 4c-Hydroxymethyl-2-benzyl-[1,3]dioxolan-2r-carbonsäure, entsprechend einer Ausbeute von 80 %, in Form farbloser Kristalle vom Schmelzpunkt 106-107°C (aus Wasser ) erhalten.

BAD ORIGINAL

Kennzahlen: SZ berechnet 236; gefunden 235

Elementaranalyse:

| | C | H |
|---|---|---|
| berechnet ($C_{12}H_{14}O_5$) | 60,50 | 5,92 |
| gefunden | 60,37 | 5,94 |

Beispiel 13

5c-Acetoxy-2-benzyl-[1,3]-dioxan-2r-carbonsäureamid

Eine Mischung von 11,85 g (0,05 Mol) 5c-Hydroxy-2-benzyl-[1,3]dioxan-2r-carbonsäureamid und 60 ml Pyridin wurde innerhalb von 30 Min.mit 51 g (0,05 Mol) Acetanhydrid versetzt und danach 1 Std. auf 50°C erwärmt. Nach 24 Std. Aufbewahren bei Raumtemperatur wurde am Rotationsverdampfer auf 20 ml eingeengt. Der Rückstand wurde mit 100 ml Diethylether versetzt. Die abgeschiedenen Kristalle wurden abfiltriert und mit Ether gewaschen. Es wurden 13 g 5c-Acetoxy-2-benzyl-[1,3]dioxan-2r-carbonsäureamid, entsprechend einer Ausbeute von 93 %, in Form farbloser Kristalle vom Schmelzpunkt 105-106°C erhalten.

$^1$H-NMR (DMSO-$d_6$): $\delta$ = 1,99 (s, 3H); 3,07 (s, 3H); 3,60/4,08 (AB-System, 2H/2H); 4,64 (m, 1H); 7,24 (m, 5H); 7,41 (breites s, 2H)

Massenspektrum: m/e 235 ($M^+$-44)

Elementaranalyse:

| | C | H | N |
|---|---|---|---|
| berechnet ($C_{14}H_{17}NO_5$) | 60,21 | 6,14 | 5,02 |
| gefunden | 60,82 | 6,06 | 5,01 |

BAD ORIGINAL

Beispiel 14

5c-n-Dodecanoyloxy-2-benzyl-[1,3]dioxan-2r-carbonsäureamid

Eine Suspension von 4,75 g (0,02 Mol) 5c-Hydroxy-2-benzyl-[1,3]dioxan-2r-carbonsäureamid in 40 ml Pyridin wurde unter Eiskühlung tropfenweise mit einer Lösung von 4,4 g (0,02 Mol) Dodecanoylchlorid in 30 ml Chloroform versetzt. Nach 24 Std. Aufbewahren bei Raumtemperatur wurde am Rotationsverdampfer auf 10 ml eingeengt. Der Rückstand wurde mit 50 ml Wasser versetzt und anschließend mit Diethylether extrahiert. Die Etherauszüge wurden nach Trocknen über Natriumsulfat eingedampft. Es wurden 7,5 g 5c-n-Dodecanoyloxy-2-benzyl-[1,3]dioxan-2r-carbonsäureamid, entsprechend einer Ausbeute von 90 %, in Form farbloser Kristalle von Schmelzpunkt 91-92°C ( aus n-Hexan) erhalten.

Elementaranalyse:

| | C | H | N |
|---|---|---|---|
| berechnet ($C_{24}H_{37}NO_5$) | 68,70 | 8,89 | 3,34 |
| gefunden | 68,40 | 9,16 | 3,20 |

Beispiel 15

5c-n-Decanoyloxy-2-benzyl-[1,3]dioxan-2r-carbonsäureamid

Eine Suspension von 9,5 g (0,04 Mol) 5c-Hydroxy-2-benzyl-[1,3]dioxan-2r-carbonsäureamid in 40 ml Pyridin wurde bei 0°C tropfenweise mit einer Lösung von 8,5 g (0,044 Mol) n-Decanoylchlorid in 50 ml Chloroform versetzt. Danach wurde 1 Std. auf 50°C erwärmt. Die nunmehr klare Lösung wurde auf 15 ml eingeengt und nach Versetzen mit 80 ml Eiswasser mit 500 ml Diethylether extrahiert. Die Etherextrakte wurden mit 0,1 n Natriumhydroxidlösung behandelt, neutral gewaschen und über Natriumsulfat getrock-

BAD ORIGINAL

net. Nach Eindampfen und Kühlen auf 0°C wurden 13,5 g 5c-n-Decanoyloxy-2-benzyl-[1,3]dioxan-2r-carbonsäureamid, entsprechend einer Ausbeute von 86 %, in Form farbloser Kristalle vom Schmelzpunkt 91,5°C ( aus n-Hexan) erhalten.

Elementaranalyse:

| | C | H | N |
|---|---|---|---|
| berechnet ($C_{22}H_{33}NO_5$) | 67,49 | 8,50 | 3,58 |
| gefunden | 67,66 | 8,11 | 3,49 |

Beispiel 16

5c-n-Octanoyloxy-2-benzyl-[1,3]dioxan-2r-carbonsäureamid

5c-Hydroxy-2-benzyl-[1,3]dioxan-2r-carbonsäureamid wurde analog Beispiel 15 mit n-Octanoylchlorid umgesetzt. Nach Aufarbeitung wurde 5c-n-Octanoyloxy-2-benzyl-[1,3]dioxan-2r-carbonsäureamid in einer Ausbeute von 90 % als farblose Kristalle ( aus n-Hexan) von Schmelzpunkt 91°C erhalten.

Elementaranalyse:

| | N |
|---|---|
| berechnet ( $C_{20}H_{29}NO_5$ ) | 3,85 |
| gefunden | 3,67 |

Beispiel 17

5c-n-Octadecanoyl-2-benzyl-[1,3]dioxan-2r-carbonsäureamid

Aus n-Octadecanoylchlorid und 5c-Hydroxy-2-benzyl-[1,3]dioxan-2r-carbonsäureamid wurde entsprechend der Arbeitsweise nach Beispiel 15 reines 5c-n-Octadecanoyl-2-benzyl-[1,3]dioxan-2r-carbonsäureamid in Form farbloser Kristalle vom Schmelzpunkt 93°C erhalten.

Elementaranalyse:

| | N |
|---|---|
| berechnet ($C_{30}H_{49}NO_5$) | 2,78 |
| gefunden | 2,82 |

BAD ORIGINAL

**Beispiel 18**

5c-Acetylsalicyloyloxy-2-benzyl-[1,3]dioxan-2r-carbonsäureamid

Eine Lösung von 5,9 g (0,025 Mol) 5c-Hydroxy-2-benzyl-[1,3]dioxan-2r-carbonsäureamid in 50 ml Pyridin wurde bei 30°C tropfenweise mit einer Mischung aus 4,95 g (0,025 Mol) Acetylsalicyloylchlorid versetzt. Es wurde eine klare Lösung erhalten. Nach 24 Std. Aufbewahren bei Raumtemperatur wurde am Rotationsverdampfer auf 15 ml eingedampft. Der Rückstand wurde mit 30 ml Wasser versetzt und dann mit 250 ml Chloroform extrahiert. Die Chloroformauszüge wurden zunächst mit 20 ml 0,1 n Kalilauge, dann mit Wasser extrahiert und schließlich über Natriumsulfat getrocknet. Der nach Abdestillieren des Lösungsmittels erhaltene Rückstand wurde in 40 ml Diethylether aufgenommen und bei 0°C zur Kristallisation gebracht.

Es wurden 8 g 5c-Acetylsalicyloyloxy-2-benzyl-[1,3]dioxan-2r-carbonsäureamid, entsprechend einer Ausbeute von 80 %, in Form farbloser Kristalle vom Schmelzpunkt 124-125°C ( aus Ethanol/Diethylether) erhalten.

$^1$H-NMR ($CDCl_3$): $\delta$ = 2,30 (s, 3H); 3,14 (s, 2H); 3,78/4,21/5,07 (ABM-System, 2H/2H/1H); 6,18 (breites s, 2H); 7,24 (m, 5H); 7,01-7,95 (ABCD, 4H)

Elementaranalyse:

| | C | H | N |
|---|---|---|---|
| berechnet ( $C_{21}H_{21}NO_7$ ) | 63,15 | 5,30 | 3,51 |
| gefunden | 63,15 | 5,57 | 3,69 |

BAD ORIGINAL

Beispiel 19

5c-Nicotinoyloxy-2-benzyl-[1,3]dioxan-2r-carbonsäureamid

6,4 g (0,0525 Mol) Nicotinsäure wurden mittels Thionylchlorid in Nicotinoylchlorid-hydrochlorid überführt. Die nach Behandeln mit 10 ml Pyridin bei 90°C erhaltene Lösung des Säurechlorids wurde bei 0°C tropfenweise mit einer Lösung von 11,8 g (0,05 Mol) 5c-Hydroxy-2-benzyl-[1,3]dioxan-2r-carbonsäureamid in 100 ml Pyridin versetzt. Nach 24 Std. Aufbewahren bei Raumtemperatur wurde das überschüssige Pyridin am Rotationsverdampfer abgedampft. Der Rückstand wurde in 100 ml Chloroform aufgenommen. Die Chloroformlösung wurde bei 10°C mit 40 ml Wasser, dann mit 25 ml 0,5 n Kalilauge und schließlich mit Wasser extrahiert. Die organische Phase wurde über Natriumsulfat getrocknet, eingedampft und mit 30 ml Diethylether versetzt. Es wurden 12,5 g 5c-Nicotinoyloxy-2-benzyl-[1,3]dioxan-2r-carbonsäureamid, entsprechend einer Ausbeute von 74 %, in Form farbloser Kristalle vom Schmelzpunkt 124-125°C erhalten.

Massenspektrum: m/e 298 ($M^+$-44)

Elementaranalyse:

| | C | H | N |
|---|---|---|---|
| berechnet ($C_{18}H_{18}N_2O_5$) | 63,15 | 5,30 | 8,18 |
| gefunden | 63,17 | 5,53 | 8,02 |

Beispiel 20

5c-Hydroxy-2-isopropyl-[1,3]dioxan-2r-carbonsäureamid

1-Isopropyl-2,5,7-trioxa-bicyclo[2.2.2]octan-6-on wurde entsprechend a) Beispiel 8 und b) Beispiel 9 mit Ammoniak umgesetzt. Nach entsprechender Aufarbeitung wurde reines 5c-Hydroxy-2-isopropyl-[1,3]dioxan-2r-carbonsäureamid

BAD ORIGINAL

in hoher Ausbeute als farblose Kristalle vom Schmelzpunkt 184-185°C ( aus wenig Methanol nach Zusatz von Diethylether/Petrolether ) erhalten.

Elementaranalyse:

| | C | H | N |
|---|---|---|---|
| berechnet ( $C_8H_{15}NO_5$) | 50,78 | 7,99 | 7,40 |
| gefunden | 51,05 | 7,80 | 7,14 |

Beispiel 21

5c-Hydroxy-2-sec-butyl-[1,3]dioxan-2r-carbonsäureamid

1-sec-Butyl-2,5,7-trioxa-bicyclo[2.2.2]octan-6-on wurde entsprechend Beispiel 8 mit Ammoniak umgesetzt. Nach Aufarbeitung wurde reines 5c-Hydroxy-2-sec-butyl-[1,3]dioxan-2r-carbonsäureamid in guter Ausbeute als farblose Kristalle vom Schmelzpunkt 161,5°C ( aus Methanol/Diethylether ) erhalten.

Elementaranalyse:

| | C | H | N |
|---|---|---|---|
| berechnet ($C_9H_{17}NO_4$) | 53,19 | 8,43 | 6,89 |
| gefunden | 53,36 | 7,97 | 6,73 |

Beispiel 22

5c-Hydroxy-2-isobutyl-[1,3]dioxan-2r-carbonsäureamid

1-Isobutyl-2,5,7-trioxa-bicyclo[2.2.2]octan-6-on wurde entsprechend Beispiel 9 mit Ammoniak umgesetzt. Nach Aufarbeitung wurde reines 5 c-Hydroxy-2-isobutyl-[1,3]dioxan-2r-carbonsäureamid in guter Ausbeute als farblose Kristalle vom Schmelzpunkt 168°C ( aus Ethylacetat ) erhalten.

IR (KBr): 3470, 3230 ,1670 $cm^{-1}$

$^1$H-NMR ( DMSO-$d_6$): $\delta$ = 0,88 (d, 6H); 1,53/1,83 ($A_2B$-System, 3H); 3,22-3,90 (m, 5H); 5,01 (d, 1H); 7,30 (s, breit 1H) 7,41 (s, breit, 1H)

BAD ORIGINAL

Massenspektrum: m/e 159 ($M^+$-44)
Elementaranalyse:

| | C | H | N |
|---|---|---|---|
| berechnet ($C_9H_{17}NO_4$) | 53,19 | 8,43 | 6,89 |
| gefunden | 53,33 | 8,64 | 6,96 |

Beispiel 23

4c-Hydroxymethyl-2-sec-butyl-[1,3]dioxolan-2r-carbonsäureamid

Methylethylbrenztraubensäure wurde, entsprechend Beispiel 6 mit Glycerin umgesetzt. Der nach Abtrennung der Hauptmenge an 1-sec-Butyl-2,5,7-trioxa-bicyclo[2.2.2]octan-6-on erhaltene Rückstand enthielt 1-sec-Butyl-2,5,7-trioxa-bicyclo-[2.2.2]-octan-6-on und 5-sec-Butyl-3,6,8-trioxa-bicyclo-[3.2.1]-octan-4-on und wurde entsprechend Beispiel 8 mit Ammoniaklösung behandelt. Die so entstandenen isomeren Carbonsäureamide wurden durch fraktionierte Kristallisation aus Ethylacetat getrennt.

Neben 5c-Hydroxy-2-sec-butyl-[1,3]dioxan-2r-carbonsäureamid wurde so in geringer Ausbeute reines 4c-Hydroxymethyl-2-sec-butyl-[1,3]dioxolan-2r-carbonsäureamid in Form farbloser Kristalle vom Schmelzpunkt 128-130°C erhalten.
Elementaranalyse:

| | C | H | N |
|---|---|---|---|
| berechnet ($C_9H_{17}NO_4$) | 53,19 | 8,43 | 6,89 |
| gefunden | 53,13 | 8,50 | 6,60 |

$^1$H-NMR (DMSO-d): $\delta$ = 0,79-0,92 (m, 6H); 1,35-2,18 (m, 3H) 3,4-4,17 (m, 5H); 5,03 (t, 1H); 7,32 (s breit, 2H)

Dr.La/Ra

BAD ORIGINAL

## Patentansprüche

1. 2,5,7-Trioxa-bicyclo [2.2.2] octan-6-one der Formel

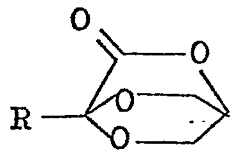

I

und 3,6,8-Trioxa-bicyclo [3.2.1] octan-4-one der Formel

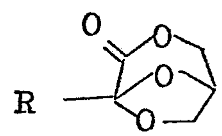

II

worin R einen verzweigten oder unverzweigten, gegebenenfalls mit weiteren Gruppen versehenen Alkylrest mit 2 bis 18 Kohlenstoffatomen, einen araliphatischen oder aromatischen Rest, der gegebenenfalls mit weiteren Gruppen substituiert ist, bedeutet sowie 1,3-Dioxan-2-carbonsäurederivate der Formel

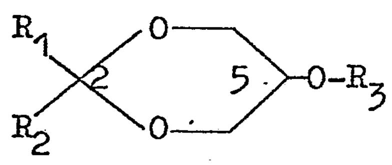

III

und 1,3-Dioxolan-2-carbonsäurederivate der Formel

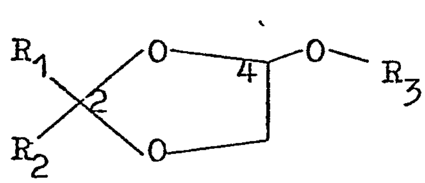

IV

worin $R_1$ einen verzweigten oder unverzweigten, gegebenenfalls mit weiteren Gruppen versehenen Alkylrest mit 1 bis 18, vorzugsweise mit 2 bis 8 Kohlenstoffatomen, einen araliphatischen oder aromatischen Rest,

der gegebenenfalls mit weiteren Gruppen substituiert ist, $R_2$ eine Carboxylgruppe, deren Wasserstoffatom gegebenenfalls durch ein Metallatom ersetzt sein kann, oder eine Carbamoylgruppe und $R_3$ Wasserstoff oder den Carbonsäurerest einer physiologisch akzeptablen Carbonsäure oder den Carbonsäurerest eines pharmakologischen Wirkstoffs bedeuten.

2. Verbindungen gemäß Anspruch 1, in denen die Reste $R_1$ + R dem Rest einer essentiellen Aminosäurevorstufe der Formel R-CO-COOH entsprechen.

3. Verbindungen nach einem der Ansprüche 1 oder 2, in denen die Reste $R_1$ + R eine Isopropyl-, eine sek.-Butyl-, eine Isobutyl-, eine Benzyl- oder eine Phenylgruppe bedeuten.

4. Verbindungen nach einem oder mehreren der Ansprüche 1 bis 3, in denen der Rest $R_3$ einen Fettsäurerest mit 2 bis 18 Kohlenstoffatomen bedeutet.

5. Verbindungen nach einem oder mehreren der Ansprüche 1 bis 3, in denen der Rest $R_3$ den Rest eines Pharmakons mit entzündungshemmender Wirkung wie beispielsweise den Acetylsalicyloyl-, den Indomethacoyl-, den 2-(3-Phenoxyphenyl)-propionoyl-Rest bedeutet.

6. Verbindungen nach einem oder mehreren der Ansprüche 1 bis 3, in denen der Rest $R_3$ den Nicotinoyl-Rest bedeutet.

7. Verfahren zur Herstellung der Verbindungen der Formeln I oder II nach einem oder mehreren der Ansprüche 1 bis 3, dadurch gekennzeichnet, daß eine α-Ketocarbonsäure der Formel

R-CO-COOH

in der R die angegebene Bedeutung besitzt, oder deren Ester, Ketale, Ketalester, Halogenide oder vergleichbare Derivate mit Glycerin oder ein den Glycerinrest lieferndes Glycerinderivat bei einer eine ausreichende Reaktionsgeschwindigkeit auslösenden Temperatur unterhalb von 160°C und in Anwesenheit von sauren Katalysatoren umgesetzt wird und das so erhaltene Gemisch aus 2,5,7-Trioxa-bicyclo [2.2.2] octan-6-on der Formel

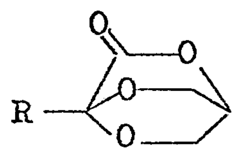

und aus 3,6,8-Trioxa-bicyclo [3.2.1] -octan-4-on der Formel

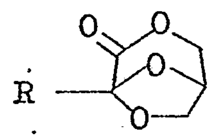

gegebenenfalls in die reinen Komponenten zerlegt wird.

8. Verfahren nach Anspruch 7, dadurch gekennzeichnet, daß als Katalysator Schwefelsäure, p-Toluolsulfonsäure oder Phosphorsäure verwendet wird.

9. Verfahren nach einem der Ansprüche 7 oder 8, dadurch gekennzeichnet, daß der Katalysator in einer Konzentration von 0,01 bis 3 Gew.-% verwendet wird.

10. Verfahren nach einem der Ansprüche 7 bis 9, dadurch gekennzeichnet, daß die Reaktion unter Abdestillieren des Reaktionswassers bzw. des aus den eingesetzten Estern oder Ketalen abgespaltenen niedrigmolekularen Alkohols, gegebenenfalls unter Mitwirkung eines inerten Schleppmittels, durchgeführt wird.

BAD ORIGINAL

11. Verfahren nach einem der Ansprüche 7 bis 9, dadurch gekennzeichnet, daß die Reaktion in Gegenwart wasserbindender Agentien durchgeführt wird.

12. Verfahren nach Anspruch 11, dadurch gekennzeichnet, daß die Reaktion in Gegenwart von Thionylchlorid durchgeführt wird.

13. Verfahren zur Herstellung von Verbindungen der Formeln III oder IV gemäß einem oder mehreren der Ansprüche 1 bis 6, dadurch gekennzeichnet, daß 2,5,7-Trioxa-bicyclo[2.2.2] octan-6-one der Formel

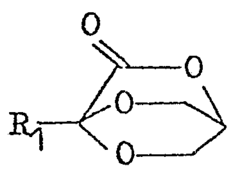

I oder/und

3,6,8-Trioxabicyclo [3.2.1] octan-4-one der Formel

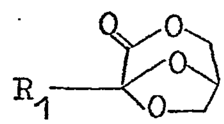

II

in 1,3-Dioxan-2-carbonsäure-Derivate der Formel

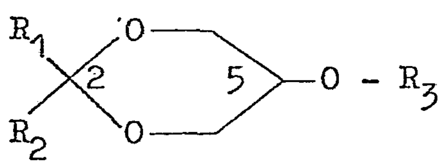

bzw. 1,3-Dioxolan-2-carbonsäure-Derivate der Formel

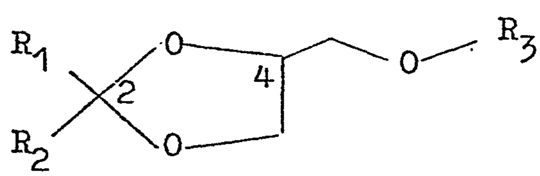

umgewandelt werden, in denen $R_1$, $R_2$ und $R_3$ die oben angegebene Bedeutung haben,

a) durch Behandlung mit basisch reagierenden Verbindungen, vorzugsweise mit Alkalihydroxiden, oder

b) durch Behandlung mit basisch reagierenden Verbindungen, vorzugsweise mit Alkalihydroxiden, und nachfolgender Freisetzung der Oxycarbonsäure mittels Mineralsäure oder

c) durch Behandlung mit Ammoniak oder

d) durch Umsetzung eines nach a), b) oder c) erhaltenen Produktes mit einer physiologisch akzeptablen Carbonsäure bzw. einem geeigneten Carbonsäurederivat oder mit einem eine Carboxylgruppe aufweisenden pharmakologischen Wirkstoff bzw. einem entsprechenden Derivat des Wirkstoffs.

14. Verfahren nach Anspruch 13, dadurch gekennzeichnet, daß die Umsetzung nach Variante d) bei einer Temperatur unter 80°C, vorzugsweise unter 60°C, entweder mit der freien Carbonsäure in Gegenwart von wasserbindenden Mitteln wie Dicyclohexylcarbodiimid oder mit reaktiven Carbonsäurederivaten wie Säurehalogeniden oder Säureanhydriden durchgeführt wird.

15. Verwendung der Stoffe nach Anspruch 1 als Arzneimittel der Human- und Tiermedizin sowie als Ernährungsbestandteil für Mensch, Tier und Mikroorganismen.

Dr.La/Ra